# EUROPEAN PATENT SPECIFICATION

(11) **EP 4 491 620 B1**
(45) Date of publication and mention of the grant of the patent: **24.06.2026**
(21) Application number: 23766039.4
(22) Date of filing: 08.03.2023
(51) Int. Cl.: C07F 9/6558, A61K 31/675, A61P 35/00

(54) **CRYSTAL FORM OF ISOCHROMAN COMPOUND**
KRISTALLFORM EINER ISOCHROMANVERBINDUNG
FORME CRISTALLINE D'UN COMPOSÉ ISOCHROMANE

(30) Priority: 10.03.2022 CN 202210234527
(43) Date of publication of application: 15.01.2025
(73) Proprietor: Zhejiang Yangli Pharmaceutical Technology Co., Ltd., Hangzhou, Zhejiang 310015 (CN)
(72) Inventor: CAI, Zhe, Shanghai 200131 (CN); SUN, Fei, Shanghai 200131 (CN); DING, Charles Z., Shanghai 200131 (CN)
(74) Representative: Dehns
(86) International application number: PCT/CN2023/080261
(87) International publication number: WO 2023/169462

(56) References cited:
- WO-A1-2021/068952
- WO-A1-2021/068952
- WO-A1-2022/057838
- CN-A- 107 530 556
- CN-A- 108 290 911
- DATABASE CAPLUS [online] 1 January 2021 (2021-01-01), MEDSHINE DISCOVERY INC. ET AL: "Benzodihydropyran compound targeting aldo-keto reductase 1c3 - WO 2021068952 A1", XP093258318, Database accession no. 2021:861960

## Description

The present application claims the right of the priority of Chinese patent application No. 2022102345275 filed on March 10, 2022.

### TECHNICAL FIELD

The present disclosure relates to a crystal form of an isochroman compound and a preparation method therefor, specifically to a compound of formula (I) and a use thereof for treating liver cancer, prostate cancer, pancreatic cancer, and/or T-cell acute lymphoblastic leukemia.

### BACKGROUND

Malignant tumor is a major disease that seriously threatens human life and health. Current treatment methods mainly include surgical therapy, chemotherapy, targeted therapy, etc. Chemotherapy is a treatment method that uses chemical drugs to kill tumor cells and inhibit the growth of tumor cells, which is a systemic treatment method. Due to the heterogeneity of malignant tumors, chemotherapy remains an important method for the treatment of tumors. However, it is this systemic treatment that causes chemotherapy to have significant side effects. There is a huge unmet clinical need for developing targeted chemotherapeutics.

Aldo-keto reductase (AKR1C3) is a member of the aldo-keto reductase family, which is mainly involved in hormone synthesis and toxin removal. Overexpression of AKR1C3 can be induced by factors such as smoking, alcohol, hepatitis B or hepatitis C infection. AKR1C3 is overexpressed in a variety of refractory cancers, such as liver cancer, lung cancer, gastric cancer, esophageal cancer, colorectal cancer, prostate cancer, and acute lymphoblastic leukemia, especially liver cancer, which has a high expression rate of more than 60%.

Currently, AKR1C3 inhibitor drugs are being developed clinically, but no good progress has been made. Taiwan OBI Pharma, Inc. reported a compound OBI-3424 targeting the AKR1C3 enzyme. OBI-3424 is a selective prodrug that releases a potent DNA alkylating agent in tumor cells with high expression of AKR1C3 enzyme, selectively kills tumor cells with high expression of AKR1C3, and enables the chemical drug to have obvious targeting effects.

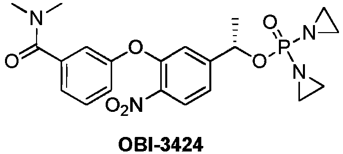

At present, the research on this target is still in the early stage. Only OBI-3424 has entered the clinical phase 1/2, the indications are mainly hepatocellular carcinoma (HCC), castration-resistant prostate cancer (CRPC), pancreatic cancer, T-cell acute lymphoblastic leukemia (T-ALL), etc, and the effectiveness and safety of OBI-3424 are still being verified. Therefore, more exploration and research are needed in this field.

WO 2021/068952A1 discloses a benzodihydropyran compound targeting AKR1C3 enzyme (aldo-keto reductase 1C3). In particular, it discloses a compound represented by formula (II) or a pharmaceutically acceptable salt or isomer thereof:

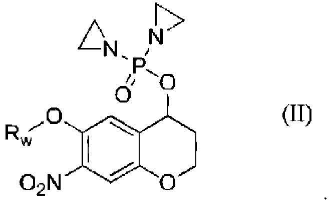

### CONTENT OF THE PRESENT INVENTION

A crystal form A of a compound of formula (I)

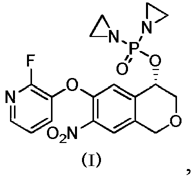

wherein the crystal form A of the compound of formula (I) has an X-ray powder diffraction pattern comprising characteristic diffraction peaks at the following 2θ angles: 8.25 ± 0.20°, 19.21 ± 0.20°, and 21.76 ± 0.20° using Cu-Kα radiation.

In some embodiments of the present disclosure, the X-ray powder diffraction pattern of the crystal form A of the compound of formula (I) comprises characteristic diffraction peaks at the following 2θ angles: 8.25 ± 0.20°, 13.27 ± 0.20°, 15.19 ± 0.20°, 16.43 ± 0.20°, 17.94 ± 0.20°, and 19.21 ± 0.20°.

In some embodiments of the present disclosure, the X-ray powder diffraction pattern of the crystal form A of the compound of formula (I) comprises characteristic diffraction peaks at the following 2θ angles: 8.25 ± 0.20°, 13.27 ± 0.20°, 15.19 ± 0.20°, 16.43 ± 0.20°, 17.94 ± 0.20°, 19.21 ± 0.20°, and 21.76 ± 0.20°.

In some embodiments of the present disclosure, the X-ray powder diffraction pattern of the crystal form A of the compound of formula (I) comprises characteristic diffraction peaks at the following 2θ angles: 8.25 ± 0.20°, 15.19 ± 0.20°, 16.43 ± 0.20°, 17.94 ± 0.20°, 19.21 ± 0.20°, 21.76 ± 0.20°, 23.56 ± 0.20°, and 26.66 ± 0.20°.

In some embodiments of the present disclosure, the X-ray powder diffraction pattern of the crystal form A of the compound of formula (I) comprises characteristic diffraction peaks at the following 2θ angles: 8.25 ± 0.20°, 12.56 ± 0.20°, 12.89 ± 0.20°, 13.27 ± 0.20°, 15.19 ± 0.20°, 16.01 ± 0.20°, 16.43 ± 0.20°, 17.60 ± 0.20°, 17.94 ± 0.20°, 19.21 ± 0.20°, 19.91 ± 0.20°, 20.21 ± 0.20°, 20.52 ± 0.20°, 21.25 ± 0.20°, 21.76 ± 0.20°, 23.02 ± 0.20°, 23.56 ± 0.20°, 24.71 ± 0.20°, 25.12 ± 0.20°, 25.88 ± 0.20°, 26.66 ± 0.20°, 27.10 ± 0.20°, 27.43 ± 0.20°, 27.84 ± 0.20°, 28.38 ± 0.20°, 29.13 ± 0.20°, 30.01 ± 0.20°, 30.83 ± 0.20°, 31.69 ± 0.20°, 32.26 ± 0.20°, 33.35 ± 0.20°, 35.01 ± 0.20°, 35.52 ± 0.20°, 36.51 ± 0.20°, 37.70 ± 0.20°, and 39.24 ± 0.20°.

In some embodiments of the present disclosure, the X-ray powder diffraction pattern of the crystal form A of the compound of formula (I) comprises characteristic diffraction peaks at the following 2θ angles: 8.25°, 12.56°, 12.89°, 13.27°, 15.19°, 16.01°, 16.43°, 17.60°, 17.94°, 19.21°, 19.91°, 20.21°, 20.52°, 21.25°, 21.76°, 23.02°, 23.56°, 24.71°, 25.12°, 25.88°, 26.66°, 27.10°, 27.43°, 27.84°, 28.38°, 29.13°, 30.01°, 30.83°, 31.69°, 32.26°, 33.35°, 35.01°, 35.52°, 36.51°, 37.70°, and 39.24°.

In some embodiments of the present disclosure, the X-ray powder diffraction pattern of the crystal form A of the compound of formula (I) comprises characteristic diffraction peaks at the following 2θ angles: 8.25 ± 0.20°, 19.21 ± 0.20°, and 21.76 ± 0.20°, and further comprises characteristic diffraction peaks at and/or 12.56 ± 0.20°, and/or 12.89 ± 0.20°, and/or 13.27 ± 0.20°, and/or 15.19 ± 0.20°, and/or 16.01 ± 0.20°, and/or 16.43 ± 0.20°, and/or 17.60 ± 0.20°, and/or 17.94 ± 0.20°, and/or 19.91 ± 0.20°, and/or 20.21 ± 0.20°, and/or 20.52 ± 0.20°, and/or 21.25 ± 0.20°, and/or 23.02 ± 0.20°, and/or 23.56 ± 0.20°, and/or 24.71 ± 0.20°, and/or 25.12 ± 0.20°, and/or 25.88 ± 0.20°, and/or 26.66 ± 0.20°, and/or 27.10 ± 0.20°, and/or 27.43 ± 0.20°, and/or 27.84 ± 0.20°, and/or 28.38 ± 0.20°, and/or 29.13 ± 0.20°, and/or 30.01 ± 0.20°, and/or 30.83 ± 0.20°, and/or 31.69 ± 0.20°, and/or 32.26 ± 0.20°, and/or 33.35 ± 0.20°, and/or 35.01 ± 0.20°, and/or 35.52 ± 0.20°, and/or 36.51 ± 0.20°, and/or 37.70 ± 0.20°, and/or 39.24 ± 0.20°.

In some embodiments of the present disclosure, the analytical data of the X-ray powder diffraction pattern of the crystal form A of the compound of formula (I) are as shown in Table 1:

**Table 1**

| No. | 2θ angle (°) | d-Spacing (Å) | Relative intensity (%) | No. | 2θ angle (°) | d-Spacing (Å) | Relative intensity (%) |
|---|---|---|---|---|---|---|---|
| 1 | 8.25 | 10.72 | 100 | 19 | 25.12 | 3.55 | 3.35 |
| 2 | 12.56 | 7.05 | 11.34 | 20 | 25.88 | 3.44 | 3.12 |
| 3 | 12.89 | 6.87 | 10.78 | 21 | 26.66 | 3.34 | 16.35 |
| 4 | 13.27 | 6.67 | 15.9 | 22 | 27.10 | 3.29 | 10.49 |
| 5 | 15.19 | 5.83 | 27.06 | 23 | 27.43 | 3.25 | 3.75 |
| 6 | 16.01 | 5.54 | 10.84 | 24 | 27.84 | 3.20 | 4.6 |
| 7 | 16.43 | 5.39 | 25.56 | 25 | 28.38 | 3.14 | 4.63 |
| 8 | 17.60 | 5.04 | 11.49 | 26 | 29.13 | 3.07 | 2.97 |
| 9 | 17.94 | 4.94 | 17.67 | 27 | 30.01 | 2.98 | 1.79 |
| 10 | 19.21 | 4.62 | 57.76 | 28 | 30.83 | 2.90 | 4.44 |
| 11 | 19.91 | 4.46 | 9.49 | 29 | 31.69 | 2.82 | 2.36 |
| 12 | 20.21 | 4.39 | 11.35 | 30 | 32.26 | 2.78 | 5.73 |
| 13 | 20.52 | 4.33 | 7.08 | 31 | 33.35 | 2.69 | 3.19 |
| 14 | 21.25 | 4.18 | 3.88 | 32 | 35.01 | 2.56 | 8.19 |
| 15 | 21.76 | 4.08 | 36.94 | 33 | 35.52 | 2.53 | 2.11 |
| 16 | 23.02 | 3.86 | 6.03 | 34 | 36.51 | 2.46 | 2.58 |
| 17 | 23.56 | 3.78 | 18.45 | 35 | 37.70 | 2.39 | 2.2 |
| 18 | 24.71 | 3.60 | 8.74 | 36 | 39.24 | 2.30 | 1.72 |

.

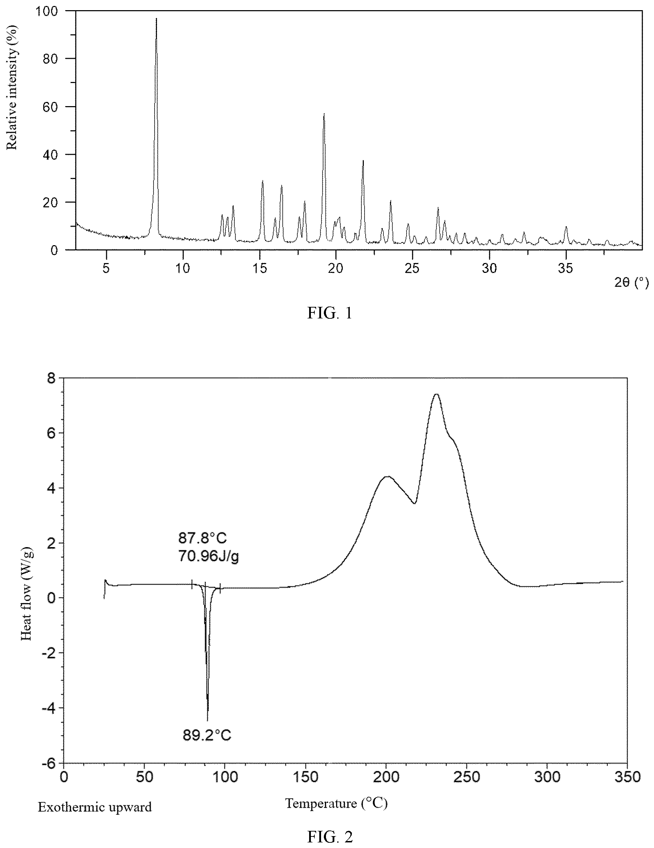

In some embodiments of the present disclosure, the crystal form A of the compound of formula (I) has an X-ray powder diffraction pattern basically as shown in FIG. 1.

In some embodiments of the present disclosure, the crystal form A of the compound of formula (I) has a differential scanning calorimetry (DSC) curve comprising an endothermic peak at 89.2 ± 3°C.

In some embodiments of the present disclosure, the crystal form A of the compound of formula (I) has a DSC pattern basically as shown in FIG. 2.

In some embodiments of the present disclosure, the crystal form A of the compound of formula (I) has a thermogravimetric analysis (TGA) curve with a weight loss of 1.03% at 100 ± 3°C.

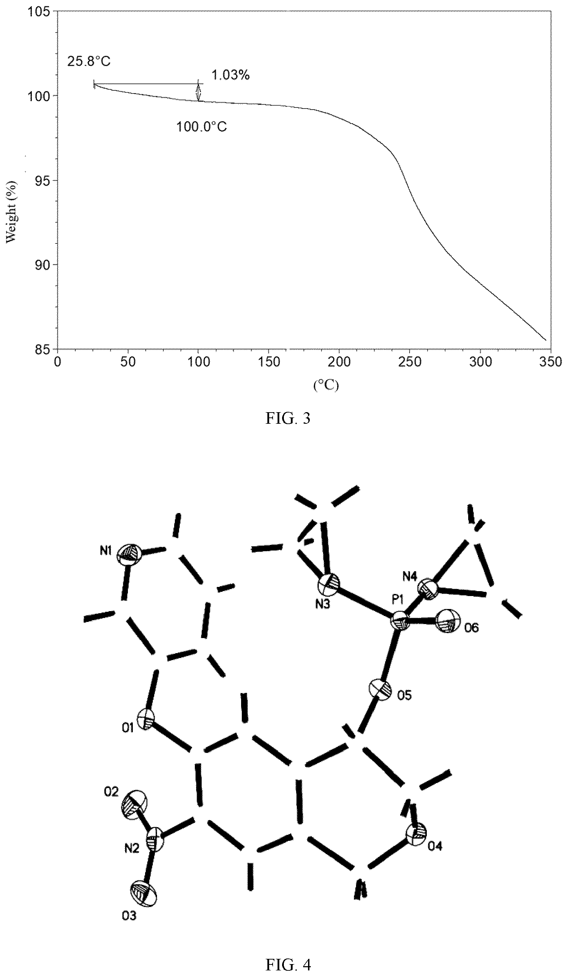

In some embodiments of the present disclosure, the crystal form A of the compound of formula (I) has a TGA pattern basically as shown in FIG. 3.

A preparation method for the crystal form A of the compound of formula (I) 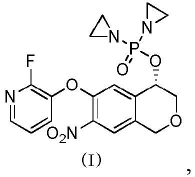 comprising: adding a compound of formula (I) in any form (crystal form or amorphous form) to a solvent, stirring at a certain temperature for a certain time, filtering, and drying the filter cake to obtain crystal form A.

In some embodiments of the present disclosure, the solvent is selected from alcohols, acetone, methyl isobutyl ketone, ethyl acetate, isopropyl acetate, tetrahydrofuran, 2-methyltetrahydrofuran, toluene, dichloromethane, 1,4-dioxane, acetonitrile, and alkanes.

In some embodiments of the present disclosure, the solvent is a mixture of any 2, 3, 4, 5, 6, 7, 8, 9, 10, 11, or 12 of alcohols, acetone, methyl isobutyl ketone, ethyl acetate, isopropyl acetate, tetrahydrofuran, 2-methyltetrahydrofuran, toluene, dichloromethane, 1,4-dioxane, acetonitrile, and alkanes.

In some embodiments of the present disclosure, the alcohols are selected from ethanol, isopropanol, and *n*-butanol.

In some embodiments of the present disclosure, the alkanes are selected from *n*-hexane, *n*-heptane, and cyclohexane.

In some embodiments of the present disclosure, the certain temperature is selected from 0°C to 65°C.

In some embodiments of the present disclosure, the certain time is selected from 1 hour to 72 hours.

In some embodiments of the present disclosure, the weight ratio of the compound of formula (I) to the solvent is selected from 1:1 to 1:30.

The present disclosure further provides crystal form A of the compound of formula (I) or the crystal form A prepared according to the above method for use in treating liver cancer, prostate cancer, pancreatic cancer, and/or T-cell acute lymphoblastic leukemia.

### Technical effect

The compound of the present disclosure has significant anti-proliferative activity on tumor cells that highly express the AKR1C3 enzyme, and the compound of the present disclosure exhibits significant anti-tumor efficacy in multiple *in vivo* efficacy models. The crystal form A of the compound of the present disclosure has a simple preparation method, good physical stability and chemical stability, and high industrial application value and economic value.

### Definition and description

Unless otherwise specified, the following terms and phrases when used herein have the following meanings. A specific phrase or term should not be considered indefinite or unclear in the absence of a particular definition, but should be understood in the ordinary sense. When a trading name appears herein, it is intended to refer to its corresponding commodity or active ingredient thereof.

The intermediate compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include, but are not limited to, the examples of the present disclosure.

The chemical reactions of the specific embodiments of the present disclosure are completed in a suitable solvent, and the solvent must be appropriate for the chemical changes of the present disclosure and the required reagents and materials thereof. In order to obtain the compounds of the present disclosure, it is sometimes necessary for those skilled in the art to modify or select the synthetic steps or reaction processes on the basis of the existing embodiments.

The present disclosure is described in detail by the examples below, but these examples do not imply any restrictions on the present disclosure which is defined by the claims.

All solvents used in the present disclosure are commercially available and can be used without further purification.

The compounds of the present disclosure are named manually or by ChemDraw^{®} software, and the commercially available compounds use the supplier catalog names.

The following abbreviations are used in the present disclosure: K₂CO₃ represents potassium carbonate; [Ru(p-cym)Cl₂]₂ represents dichloro(p-cymene)ruthenium(II) dimer; EtOH represents ethanol; MTBE represents *tert-*butyl methyl ether; DCM represents dichloromethane; PE represents petroleum ether; DIBAL-H represents diisobutylaluminum hydride; DMF represents *N,N*-dimethylformamide; DMSO represents dimethyl sulfoxide; EtOAc represents ethyl acetate; MeOH represents methanol; n-heptane represents normal heptane; SiO₂ represents 100 to 200 mesh silica gel powder, used for column chromatography; Et₃SiH represents triethylsilane; HOAc represents acetic acid; NaIO₄ represents sodium periodate; THF represents tetrahydrofuran; DIPEA represents *N,N-*diisopropylethylamine; (*S*)-CBS represents (*S*)-3,3-diphenyl-1-methylpyrrolidino[1,2-*c*]-1,3,2-oxazaborole; K₃PO₄ represents tripotassium phosphate; HP-β-CD represents cyclodextrin; QW represents frequency of administration.
X-ray powder diffraction (X-ray powder diffractometer, XRPD) method of the present disclosure
Instrument model: Dandong Haoyuan DX-2700BH X-ray diffractometer
Test method: Approximately 10 to 20 mg of sample is used for XRPD detection.
The detailed XRPD parameters are as follows:
Light tube: Cu, kα, (λ = 1.54184 Ǻ).
Light tube voltage: 40 kV, light tube current: 30 mA
Divergence slit: 1 mm
Detector slit: 0.3 mm
Anti-scatter slit: 1 mm
Scan range: 3 to 40 deg
Step size: 0.02 deg
Step time: 0.5 seconds
Differential scanning calorimetry (differential scanning calorimeter, DSC) method of the present disclosure
Instrument model: METTLER TOLEDO DSC1 differential scanning calorimeter
Test method: The sample (2 to 6 mg) is taken and placed in a 30 µL DSC gold-plated high-pressure crucible for testing, and the sample is heated from 40°C to 350°C at a heating rate of 10°C/min.
Thermogravimetric analysis (thermal gravimetric analyzer, TGA) method of the present disclosure
Instrument model: TA TGA550 thermogravimetric analyzer
Test method: The sample (2 to 10 mg) is taken and placed in an aluminum crucible, and placed in a platinum basket for testing. The sample is heated from 40°C to 500°C under nitrogen (N₂) conditions at a gas flow rate of 40 mL/min with a heating rate of 10°C/min.
The parameter of the single crystal X-ray diffraction detection instrument of the present disclosure
Instrument model: Bruker D8 VENTURE CMOS Photon II diffractometer.
Cryogenic system: Oxford Cryostream 800
Light source: Cu: λ = 1.54184 Å, 2.5 kW,
distance from crystal to detector: d = 45 mm
Tube voltage: 50 kV
Tube current: 50 mA.

### BRIEF DESCRIPTION OF THE DRAWINGS

FIG. 1 is an XRPD pattern with Cu-Kα radiation of the crystal form A of the compound of formula (I);
FIG. 2 is a DSC pattern of the crystal form A of the compound of formula (I);
FIG. 3 is a TGA pattern of the crystal form A of the compound of formula (I);
FIG. 4 is an ellipsoid diagram of the single crystal X-ray diffraction three-dimensional structure of the compound of formula (I);
FIG. 5 is the tumor growth signal - time curve;
FIG. 6 is a schematic diagram of tumor weight at the end point of the experiment;
FIG. 7 is the animal weight - time curve.

### DETAILED DESCRIPTION OF THE PREFERRED EMBODIMENT

The present disclosure is described in detail by the examples below, but it does not mean that there are any adverse restrictions on the present disclosure. The crystal form of the compound of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure which is defined by the claims.

### Example 1: Preparation of compound of formula (I)

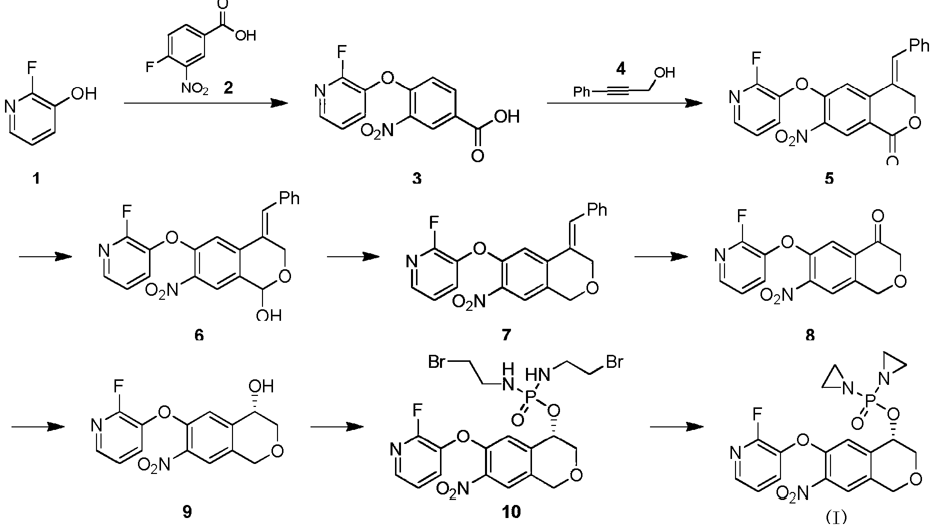

Step A: DMF (30 L) was added to a reaction kettle, and the mechanical stirring was started. Then starting material 1 (4 kg, 35.37 mol) and starting material 2 (6.6 kg, 35.71 mol) were added thereto. After the addition was completed, the internal temperature was controlled to rise to 30 to 40°C, and K₂CO₃ (12.2 kg, 88.27 mol) was added thereto in batches. Gas was generated, and the internal temperature was controlled at 40 to 50°C. After the addition was completed, the reaction mixture was stirred at 45 to 55°C for 16 hours. 42 L of ice water was added to the reaction mixture, and 42 L of 4 mol/L hydrochloric acid was slowly added thereto with stirring. A large amount of yellow solid was precipitated, and the mixture was filtered under reduced pressure, then the filter cake was washed with 45 L of water. The filter cake was dried under vacuum to obtain intermediate 3. ¹H NMR (400 MHz, DMSO-d₆) δ 13.81 - 13.38 (m, 1H), 8.55 (d, *J* = 2.1 Hz, 1H), 8.24 - 8.12 (m, 2H), 7.97 (ddd, *J* = 1.6, 8.1, 10.0 Hz, 1H), 7.50 (ddd, *J* = 0.7, 4.8, 7.9 Hz, 1H), 7.29 (d, *J* = 8.7 Hz, 1H).

Step B: 1,4-dioxane (27 L) and H₂O (2.7 L) were added to a reaction kettle, then intermediate 3 (3230.60 g, 11.613 mol), intermediate 4 (3230.60 g, 11.613 mol), guanidine carbonate (1047.08 g, 5.812 mol), and HOAc (662 mL, 11.575 mol) were sequentially added thereto with stirring. Nitrogen was blown into the kettle for 15 minutes. [Ru(p-cym)Cl₂]₂ (355.37 g, 0.580 mol) was added thereto in one portion. The reaction mixture was heated to 90°C and stirred for 17 hours, and then cooled to 25°C. 2.7 L of water was added thereto. The mixture was allowed to stand for about 23 hours at about 5°C, and the solid was precipitated. The mixture was filtered, and then the filter cake was added with 10 L of EtOH/H₂O (volume ratio of 1:1), stirred at room temperature for 4 hours, and subjected to suction filtration. The filter cake was added with 8 L of EtOAc/MTBE (volume ratio of 2:1), stirred at room temperature for 17 hours, and subjected to suction filtration. The filter cake was washed with EtOAc/MTBE (volume ratio of 2:1) (1 L * 2) and the filter cake was dried under vacuum to obtain intermediate 5. ¹H NMR (400 MHz, DMSO-d₆) δ 8.64 (s, 1H), 8.18 - 8.11 (m, 1H), 7.92 (ddd, *J* = 1.4, 8.2, 10.0 Hz, 1H), 7.80 (s, 1H), 7.60 (s, 1H), 7.51 - 7.40 (m, 4H), 7.35 (d, *J* = 6.9 Hz, 2H), 5.42 (d, *J* = 0.9 Hz, 2H).

Step C: Anhydrous DCM (22 L) was added to a reaction kettle, and intermediate 5 (2200 g, 5.608 mol) was added thereto with stirring. The reaction temperature was lowered to -60 to -50°C under nitrogen atmosphere. DIBAL-H (6700 mL, 6.7 mol) was added dropwise to the kettle. After the dropwise addition was completed, the internal temperature was controlled at -60 to -50°C, and the reaction mixture was stirred for another 1.5 hours under nitrogen atmosphere. MeOH (455 mL) was added dropwise to the kettle and the internal temperature was controlled at -60 to -50°C. After the dropwise addition was completed, the internal temperature was slowly warmed to 0°C, and isopropanol (7.3 L), dichloromethane (15 L), and potassium sodium L-(+)-tartrate tetrahydrate aqueous solution (3.5 kg, 22 L aqueous solution) were sequentially added thereto. After the addition was completed, the reaction mixture was stirred at room temperature for 4 hours, and then allowed to stand for 12 hours for phase separation. The organic phase was concentrated to obtain a yellow solid and dried under vacuum to obtain intermediate 6. ¹H NMR (400 MHz, DMSO-d₆) δ 8.10 (s, 1H), 8.03 - 8.00 (m, 1H), 7.98 (s, 1H), 7.61 (ddd, *J* = 1.4, 8.2, 10.1 Hz, 1H), 7.52 (s, 1H), 7.48 - 7.41 (m, 2H), 7.40 - 7.33 (m, 4H), 7.29 (d, *J* = 5.8 Hz, 1H), 5.94 (d, *J* = 5.6 Hz, 1H), 4.94 (dd, *J* = 1.3, 14.4 Hz, 1H), 4.69 (dd, *J* = 0.9, 14.4 Hz, 1H).

Step D: DCM (9.4 L) was added to a reaction kettle, and then intermediate 6 (1880.00 g, 4.767 mol) and Et₃SiH (2284 mL, 14.300 mol) were added thereto with stirring. The nitrogen flow protection was turned on, and the internal temperature was 0 to 5°C after the addition was completed. Boron trifluoride diethyl etherate (1765 mL, 614.301 mol) was added dropwise thereto, and the internal temperature was controlled at 0 to 10°C. The reaction mixture was stirred for another 1 hour. K₂CO₃ aqueous solution (1.05 kg dissolved in 12 L of water) was added to the reaction mixture in batches. After the addition was completed, the reaction mixture was stirred for another 20 minutes and filtered under reduced pressure to obtain a filter cake. The phases of the filtrate were separated, and the obtained organic phase was concentrated to 5 L. A yellow solid was precipitated and the mixture was filtered. The obtained filter cake was combined with the previous filter cake. The filter cake was dried under vacuum to obtain intermediate 7. ¹H NMR (400 MHz, DMSO-d₆) δ 8.08 (s, 1H), 8.04 (s, 1H), 7.99 (td, *J* = 1.5, 4.8 Hz, 1H), 7.58 (ddd, *J* = 1.6, 8.2, 10.2 Hz, 1H), 7.52 (s, 1H), 7.48 - 7.40 (m, 2H), 7.38 - 7.30 (m, 4H), 4.84 (s, 2H), 4.76 (d, *J* = 1.3 Hz, 2H).

Step E: DCM (7.0 L), MeCN (7.0 L), and water (10.5 L) were added to a reaction kettle, then intermediate 7 (1740.00 g, 4.599 mol) and ruthenium trichloride trihydrate (12.03 g, 0.0460 mol) were added thereto with stirring, and the internal temperature was controlled at 10 to 20°C. NaIO₄ (2147.72 g, 14.717 mol) was added thereto in batches within 2 hours, and the internal temperature was controlled at 10 to 20°C. After the addition was completed, the reaction mixture was stirred for another 0.5 hours, added with DCM (14.0 L), stirred for 5 minutes, and then filtered through diatomite to remove excess inorganic salts. The phases of the filtrate were separated, and the organic phase was added with NaOH aqueous solution (8 L, 1 mol/L), filtered through diatomite, and then the phases were separated again. The organic phase was washed sequentially with 8 L of saturated sodium sulfite aqueous solution and 8 L of saturated brine. The organic phase was concentrated to obtain a crude product. The crude product was purified by column chromatography (SiO₂, n-heptane: EtOAc = 100:0 to 10:1) to obtain intermediate 8. ¹H NMR (400 MHz, CDCl₃) δ 8.05 (td, *J* = 1.6, 4.8 Hz, 1H), 7.76 (s, 1H), 7.52 (ddd, *J* = 1.6, 7.8, 9.5 Hz, 1H), 7.45 (s, 1H), 7.22 (ddd, *J* = 0.7, 4.8, 7.8 Hz, 1H), 4.86 (s, 2H), 4.32 (s, 2H).

Step F: 450 mL of anhydrous THF and 147.6 mL of (S)-CBS were sequentially added to a reaction kettle under nitrogen atmosphere and cooled to 0 to 10°C. 296.2 mL of borane-methyl sulfide complex (10 M) was added dropwise to the system, and the reaction was carried out for 1 hour with magnetic stirring. A solution of intermediate 8 in THF was slowly added dropwise to the system (preparation method: 450 g of intermediate 7 was weighed and dissolved in 1800 mL of THF), and the temperature was controlled at 0 to 10°C, and then the reaction was carried out for 1 hour. After the reaction was completed, 180 mL of MeOH was slowly added dropwise to quench the reaction. 2250 mL of 2.0 mol/L hydrochloric acid was measured and slowly added dropwise to the system, and a large amount of solid was precipitated. The mixture was filtered under reduced pressure and the filter cake was left for use. The phases of the filtrate were separated to obtain the organic phase, and the aqueous phase was extracted with 4500 mL of EtOAc. The organic phases were combined and concentrated under reduced pressure to solid at 35 to 40°C. The concentrate was combined with the filter cake, added with 1350 mL of ethanol/2.0 M hydrochloric acid (volume ratio of 2:1) solution, heated to 60 to 70°C and stirred for 1 hour. The reaction mixture was cooled to 20°C to 25°C, filtered under reduced pressure, and the filter cake was rinsed with 900 mL of purified water. The filter cake (wet product) was directly added to 1350 mL of ethanol/purified water (volume ratio of 1:1) mixed solution and stirred at 20 to 25°C for 2 hours. The mixture was filtered under reduced pressure and the filter cake was rinsed with 900 mL of purified water. The filter cake was put into a baking tray of a vacuum drying oven and dried under vacuum at 40°C to obtain intermediate 9.

Step G: 137 g of intermediate 9 and 2055 mL of anhydrous THF were added to a 5 L single-layer glass reaction kettle under nitrogen atmosphere, and cooled to -40 to -30°C under magnetic stirring. The temperature of the system was controlled at -40 to -30°C, and 56.7 mL of phosphorus oxychloride was added thereto in one portion, and then 106.3 mL of 2-*tert*-butyl-1,1,3,3-tetramethylguanidine was slowly added dropwise thereto. After the dropwise addition was completed, the temperature was maintained at -40 to -30°C and the reaction was carried out for 4 to 5 hours. After the reaction was completed, the reaction mixture was cooled to -50 to -40°C, and 548 g of 2-bromoethylamine hydrobromide was added thereto in one portion, and then the temperature was controlled at -50 to -40°C and 467.3 mL of DIPEA was slowly added dropwise thereto. After the dropwise addition was completed, the temperature was maintained at -50 to -40°C and the reaction was carried out for 12 hours. The reaction was quenched by slowly adding 2055 mL of purified water dropwise to the reaction mixture. The temperature was naturally raised to 0 to 10°C, and the reaction mixture was allowed to stand for phase separation. The separated organic phase was concentrated, and the aqueous phase was extracted once with 1781 mL. The concentrate and extract were mixed and washed sequentially with 1096 mL of 0.1 M hydrochloric acid, 1096 mL of 1% K₃PO₄ aqueous solution, and 1096 mL of saturated brine. The separated organic phase was dried by adding 274 g of anhydrous sodium sulfate. The mixture was filtered under vacuum and the filter cake was rinsed with 274 mL of EtOAc. The filtrate was concentrated under reduced pressure to an oil or semi-solid. The concentrate was transferred to a reaction kettle, and 411 mL of isopropyl acetate was added thereto, heated to 75 to 80°C, and stirred to dissolve completely. Then the mixture was slowly cooled to 10°C, and a large amount of white solid was precipitated, and the mixture was crystallized for 12 hours. The mixture was filtered under vacuum and the filter cake was rinsed with 50 mL of isopropyl acetate. The filter cake was dried under vacuum to obtain intermediate 10.

Step H: 3 L of acetone and 300 g of intermediate 10 were sequentially added to a reaction kettle under nitrogen atmosphere, and 319.4 g of K₃PO₄ was added thereto with stirring. The system was heated to 35 to 45°C, stirred and reacted for 12 hours, then added with 212.9 g of K₃PO₄, stirred and reacted for another 5 hours. The reaction mixture was cooled to 20 to 25°C, filtered under vacuum, and the filter cake was rinsed with 150 mL of EtOAc. The filtrate was concentrated under reduced pressure. The K₃PO₄ filter cake was transferred to a 5 L single-layer glass reaction kettle, and 1.5 L of EtOAc was added to slurry and wash. The mixture was filtered, and the filtrate and concentrate were combined. Additional 0.75 L of EtOAc was added thereto, and 1.5 L of ice water was added for washing. The phases were separated, and the aqueous phase was extracted once with 1.5 L of EtOAc. The organic phases were combined, then washed once with 2.25 L of 10% brine, allowed to stand for phase separation, and the organic phase was separated. The organic phase was dried for 30 minutes by adding 600 g of anhydrous sodium sulfate. The mixture was filtered under vacuum to filter out anhydrous sodium sulfate and the filter cake was rinsed with 60 mL of EtOAc. The filtrate was concentrated under reduced pressure to obtain an oil. 328 mL of isopropyl acetate was quickly added to the oil, and the mixture was crystallized with magnetic stirring for 10 hours. The mixture was filtered under vacuum, and the filter cake was rinsed with mother liquor and dried under reduced pressure to obtain the compound of formula (I).

### Example 2: Preparation of crystal form A of compound of formula (I)

320 g of the compound of formula (I) and 1280 mL of isopropyl acetate were sequentially added to a 2 L single-layer glass reaction kettle, heated to 60 to 65°C under an oil bath with magnetic stirring. After the solid was dissolved completely, the mixture was filtered while hot, and the filtrate was crystallized at 20 to 25°C for 2 hours. The mixture was filtered under vacuum and the filter cake was rinsed with 120 mL of isopropyl acetate. The filter cake was dried under vacuum to obtain a white solid, which was crystal form A. For crystal form A, the XRPD pattern is shown in FIG. 1, the DSC pattern is shown in FIG. 2, and the TGA pattern is shown in FIG. 3.

### Example 3: Single crystal X-ray diffraction detection analysis of compound of formula (I)

The preparation method of the single crystal is as follows:
The compound of formula (I) (0.5 g) was dissolved in a test tube of isopropyl acetate (10 mL), and allowed to stand and slowly volatilized at 10 to 15°C while the test tube was open, and the single crystal was obtained after 20 days of cultivation. The crystal system is a monoclinic crystal with space group P2(1), and the unit cell parameters are a = 10.0634(3) Å, b = 8.8860(2) Å, c = 10.8632(3) Å, α = γ = 90, β = 95.3960(10), volume V = 967.12(4) Å3, and the absolute configuration parameter Flack value is 0.049(9). The ellipsoid diagram of the single-molecule three-dimensional structure of the compound of formula (I) is shown in FIG. 4.

**Table 2: Atomic coordinates (× 10⁴) and equivalent isotropic displacement parameters (Å² × 10³) of the crystal of the compound of formula (I)**

| | x | y | z | U(eq) |
|---|---|---|---|---|
| C(1) | 10735(2) | 6612(3) | 3103(2) | 30(1) |
| P(1) | 5478(1) | 3193(1) | 2012(1) | 22(1) |
| F(1) | 11806(2) | 5776(2) | 3487(2) | 40(1) |
| O(1) | 9972(2) | 6221(2) | 5083(2) | 34(1) |
| N(1) | 10689(2) | 7142(3) | 1979(2) | 37(1) |
| O(3) | 9462(2) | 6590(2) | 8643(2) | 44(1) |
| C(3) | 8718(2) | 7771(3) | 3534(2) | 34(1) |
| N(3) | 7043(2) | 3318(3) | 1674(2) | 29(1) |
| N(2) | 9336(2) | 6861(2) | 7538(2) | 29(1) |
| O(2) | 9758(2) | 8000(2) | 7073(2) | 46(1) |
| C(2) | 9776(2) | 6859(3) | 3919(2) | 28(1) |
| O(4) | 4502(2) | 3073(2) | 5898(1) | 30(1) |
| C(4) | 8658(3) | 8371(4) | 2349(3) | 40(1) |
| N(4) | 4601(2) | 4563(2) | 1279(2) | 27(1) |
| O(6) | 4972(2) | 1649(2) | 1828(2) | 29(1) |
| C(6) | 8859(2) | 5559(3) | 5510(2) | 25(1) |
| O(5) | 5521(2) | 3799(2) | 3386(2) | 25(1) |
| C(5) | 9645(3) | 8016(4) | 1603(2) | 40(1) |
| C(9) | 6699(2) | 4105(2) | 6441(2) | 21(1) |
| C(8) | 6996(2) | 3858(3) | 5227(2) | 22(1) |
| C(7) | 8075(2) | 4567(3) | 4775(2) | 25(1) |
| C(10) | 7512(2) | 5054(3) | 7191(2) | 23(1) |
| C(11) | 8567(2) | 5788(3) | 6725(2) | 23(1) |
| C(12) | 5474(2) | 3391(3) | 6902(2) | 26(1) |
| C(13) | 6127(2) | 2839(2) | 4382(2) | 24(1) |
| C(14) | 5047(3) | 2076(3) | 5045(2) | 32(1) |
| C(15) | 7405(3) | 3200(4) | 406(2) | 39(1) |
| C(16) | 7609(3) | 4628(3) | 1090(3) | 43(1) |
| C(17) | 3925(2) | 4162(3) | 43(2) | 31(1) |
| C(18) | 3135(3) | 4369(4) | 1092(3) | 38(1) |

**Table 3: Bond lengths [A] and bond angles [deg] of the crystal of the compound of formula (I)**

| | |
|---|---|
| C(1)-N(1) | 1.305(3) |
| C(1)-F(1) | 1.343(3) |
| C(1)-C(2) | 1.388(3) |
| P(1)-O(6) | 1.4703(18) |
| P(1)-O(5) | 1.5837(17) |
| P(1)-N(3) | 1.654(2) |
| P(1)-N(4) | 1.662(2) |
| O(1)-C(2) | 1.383(3) |
| O(1)-C(6) | 1.384(3) |
| N(1)-C(5) | 1.339(4) |
| O(3)-N(2) | 1.219(3) |
| C(3)-C(2) | 1.372(4) |
| C(3)-C(4) | 1.389(4) |
| N(3)-C(15) | 1.462(3) |
| N(3)-C(16) | 1.466(3) |
| N(2)-O(2) | 1.225(3) |
| N(2)-C(11) | 1.470(3) |
| O(4)-C(12) | 1.424(3) |
| O(4)-C(14) | 1.428(3) |
| C(4)-C(5) | 1.376(4) |
| N(4)-C(18) | 1.480(3) |
| N(4)-C(17) | 1.490(3) |
| C(6)-C(7) | 1.385(3) |
| C(6)-C(11) | 1.394(3) |
| O(5)-C(13) | 1.464(3) |
| C(9)-C(10) | 1.386(3) |
| C(9)-C(8) | 1.397(3) |
| C(9)-C(12) | 1.513(3) |
| C(8)-C(7) | 1.385(3) |
| C(8)-C(13) | 1.508(3) |
| C(10)-C(11) | 1.382(3) |
| C(13)-C(14) | 1.519(4) |
| C(15)-C(16) | 1.475(4) |
| C(17)-C(18) | 1.461(4) |
| N(1)-C(1)-F(1) | 116.5(2) |
| N(1)-C(1)-C(2) | 125.1(3) |
| F(1)-C(1)-C(2) | 118.4(2) |
| O(6)-P(1)-O(5) | 115.05(9) |
| O(6)-P(1)-N(3) | 110.91(11) |
| O(5)-P(1)-N(3) | 104.16(10) |
| O(6)-P(1)-N(4) | 117.18(11) |
| O(5)-P(1)-N(4) | 99.73(10) |
| N(3)-P(1)-N(4) | 108.57(11) |
| C(2)-O(1)-C(6) | 115.46(18) |
| C(1)-N(1)-C(5) | 116.9(2) |
| C(2)-C(3)-C(4) | 118.3(2) |
| C(15)-N(3)-C(16) | 60.48(19) |
| C(15)-N(3)-P(1) | 122.10(16) |
| C(16)-N(3)-P(1) | 124.48(19) |
| O(3)-N(2)-O(2) | 124.2(2) |
| O(3)-N(2)-C(11) | 117.6(2) |
| O(2)-N(2)-C(11) | 118.1(2) |
| C(3)-C(2)-O(1) | 124.1(2) |
| C(3)-C(2)-C(1) | 117.7(2) |
| O(1)-C(2)-C(1) | 118.1(2) |
| C(12)-O(4)-C(14) | 110.02(19) |
| C(5)-C(4)-C(3) | 119.1(3) |
| C(18)-N(4)-C(17) | 58.91(17) |
| C(18)-N(4)-P(1) | 117.26(17) |
| C(17)-N(4)-P(1) | 115.69(17) |
| O(1)-C(6)-C(7) | 120.8(2) |
| O(1)-C(6)-C(11) | 120.3(2) |
| C(7)-C(6)-C(11) | 118.7(2) |
| C(13)-O(5)-P(1) | 118.10(14) |
| N(1)-C(5)-C(4) | 122.9(2) |
| C(10)-C(9)-C(8) | 119.0(2) |
| C(10)-C(9)-C(12) | 120.97(19) |
| C(8)-C(9)-C(12) | 120.0(2) |
| C(7)-C(8)-C(9) | 120.5(2) |
| C(7)-C(8)-C(13) | 119.1(2) |
| C(9)-C(8)-C(13) | 120.3(2) |
| C(8)-C(7)-C(6) | 120.6(2) |
| C(11)-C(10)-C(9) | 120.2(2) |
| C(10)-C(11)-C(6) | 121.0(2) |
| C(10)-C(11)-N(2) | 117.7(2) |
| C(6)-C(11)-N(2) | 121.2(2) |
| O(4)-C(12)-C(9) | 110.60(17) |
| O(5)-C(13)-C(8) | 106.11(17) |
| O(5)-C(13)-C(14) | 110.06(19) |
| C(8)-C(13)-C(14) | 112.30(19) |
| O(4)-C(14)-C(13) | 111.49(19) |
| N(3)-C(15)-C(16) | 59.92(18) |
| N(3)-C(16)-C(15) | 59.60(17) |
| C(18)-C(17)-N(4) | 60.20(16) |
| C(17)-C(18)-N(4) | 60.89(16) |

**Table 4: Torsion angles [deg] of the compound of formula (I)**

| | |
|---|---|
| F(1)-C(1)-N(1)-C(5) | -177.9(2) |
| C(2)-C(1)-N(1)-C(5) | 1.5(4) |
| O(6)-P(1)-N(3)-C(15) | 70.0(3) |
| O(5)-P(1)-N(3)-C(15) | -165.7(2) |
| N(4)-P(1)-N(3)-C(15) | -60.1(3) |
| O(6)-P(1)-N(3)-C(16) | 144.1(2) |
| O(5)-P(1)-N(3)-C(16) | -91.6(2) |
| N(4)-P(1)-N(3)-C(16) | 14.0(2) |
| C(4)-C(3)-C(2)-O(1) | 178.3(2) |
| C(4)-C(3)-C(2)-C(1) | 1.0(4) |
| C(6)-O(1)-C(2)-C(3) | 46.2(3) |
| C(6)-O(1)-C(2)-C(1) | -136.5(2) |
| N(1)-C(1)-C(2)-C(3) | -2.3(4) |
| F(1)-C(1)-C(2)-C(3) | 177.1(2) |
| N(1)-C(1)-C(2)-O(1) | -179.7(2) |
| F(1)-C(1)-C(2)-O(1) | -0.4(3) |
| C(2)-C(3)-C(4)-C(5) | 0.9(4) |
| O(6)-P(1)-N(4)-C(18) | 32.8(2) |
| O(5)-P(1)-N(4)-C(18) | -91.97(19) |
| N(3)-P(1)-N(4)-C(18) | 159.41(19) |
| O(6)-P(1)-N(4)-C(17) | -33.8(2) |
| O(5)-P(1)-N(4)-C(17) | -158.59(17) |
| N(3)-P(1)-N(4)-C(17) | 92.79(18) |
| C(2)-O(1)-C(6)-C(7) | 48.7(3) |
| C(2)-O(1)-C(6)-C(11) | -136.4(2) |
| O(6)-P(1)-O(5)-C(13) | 48.70(18) |
| N(3)-P(1)-O(5)-C(13) | -72.92(17) |
| N(4)-P(1)-O(5)-C(13) | 174.97(16) |
| C(1)-N(1)-C(5)-C(4) | 0.6(4) |
| C(3)-C(4)-C(5)-N(1) | -1.8(4) |
| C(10)-C(9)-C(8)-C(7) | -0.9(3) |
| C(12)-C(9)-C(8)-C(7) | 176.6(2) |
| C(10)-C(9)-C(8)-C(13) | -179.6(2) |
| C(12)-C(9)-C(8)-C(13) | -2.0(3) |
| C(9)-C(8)-C(7)-C(6) | -1.5(3) |
| C(13)-C(8)-C(7)-C(6) | 177.2(2) |
| O(1)-C(6)-C(7)-C(8) | 177.2(2) |
| C(1 1)-C(6)-C(7)-C(8) | 2.2(3) |
| C(8)-C(9)-C(10)-C(11) | 2.5(3) |
| C(12)-C(9)-C(10)-C(11) | -175.0(2) |
| C(9)-C(10)-C(11)-C(6) | -1.8(3) |
| C(9)-C(10)-C(11)-N(2) | 175.8(2) |
| 0(1)-C(6)-C(11)-C(10) | -175.6(2) |
| C(7)-C(6)-C(11)-C(10) | -0.5(3) |
| O(1)-C(6)-C(11)-N(2) | 6.9(3) |
| C(7)-C(6)-C(11)-N(2) | -178.1(2) |
| O(3)-N(2)-C(11)-C(10) | 33.1(3) |
| O(2)-N(2)-C(11)-C(10) | -143.8(2) |
| O(3)-N(2)-C(11)-C(6) | -149.3(2) |
| O(2)-N(2)-C(11)-C(6) | 33.8(3) |
| C(14)-O(4)-C(12)-C(9) | 59.2(3) |
| C(10)-C(9)-C(12)-0(4) | 153.1(2) |
| C(8)-C(9)-C(12)-0(4) | -24.4(3) |
| P(1)-O(5)-C(13)-C(8) | 135.98(16) |
| P(1)-O(5)-C(13)-C(14) | -102.29(19) |
| C(7)-C(8)-C(13)-O(5) | -63.4(3) |
| C(9)-C(8)-C(13)-O(5) | 115.3(2) |
| C(7)-C(8)-C(13)-C(14) | 176.3(2) |
| C(9)-C(8)-C(13)-C(14) | -5.0(3) |
| C(12)-O(4)-C(14)-C(13) | -68.4(3) |
| O(5)-C(13)-C(14)-O(4) | -79.1(2) |
| C(8)-C(13)-C(14)-O(4) | 38.9(3) |
| P(1)-N(3)-C(15)-C(16) | 114.4(2) |
| P(1)-N(3)-C(16)-C(15) | -110.6(2) |
| P(1)-N(4)-C(17)-C(18) | 107.7(2) |
| P(1)-N(4)-C(18)-C(17) | -105.0(2) |

### Experimental example 1: Anti-proliferative activity of compound of formula (I) on HepG2 cell line

### Experimental materials:

DMEM medium, penicillin/streptomycin antibiotics were purchased from Wisent, and fetal bovine serum was purchased from Biosera. CellTiter-Glo (chemiluminescent cell viability assay reagent) reagent was purchased from Promega. The HepG2 cell line was purchased from the Cell Bank of the Chinese Academy of Sciences. Nivo multimode microplate reader (PerkinElmer).

### Experimental methods:

HepG2 cells (liver cancer) were seeded in a white 384-well plate with 25 µM of cell suspension per well containing 1000 HepG2 cells. The cell plate was incubated overnight in a carbon dioxide incubator. The compound to be tested was 3-fold diluted to the ninth concentration with a multi-channel pipette, that is, the compound to be tested was diluted from 200 µM to 30 nM, and an experiment for the sample assayed in duplicate was set up. 99 µM of culture medium was added to the middle plate. According to the corresponding position, 1 µM per well of the gradient-diluted compound was transferred to the middle plate, then 25 µM per well was transferred to the cell plate after mixing well. The concentration of the compound transferred to the cell plate ranged from 1 µM to 0.15 nM. The cell plate was incubated in the carbon dioxide incubator for 5 days. Another cell plate was prepared, and the signal value as the maximum value (Max value in the following equation) was read on the day of drug addition to participate in data analysis. 20 µM of chemiluminescent cell viability assay reagent was added to each well of the cell plate, and the plate was incubated at room temperature for 10 minutes to stabilize the luminescent signal. A multimode microplate reader was used for reading. 20 µM of chemiluminescent cell viability assay reagent was added to each well of the cell plate, and the plate was incubated at room temperature for 10 minutes to stabilize the luminescent signal. A multimode microplate reader was used for reading.

### Data analysis:

The equation (Sample - Min)/(Max - Min) * 100% was used to convert the raw data into the inhibition rate, and the value of IC₅₀ could be obtained by curve fitting with four parameters ("log(inhibitor) vs. response -- Variable slope" mode in GraphPad Prism). Table 5 provides the inhibitory activity of the compound of formula (I) on the proliferation of HepG2 cells.

**Table 5: Data of anti-proliferative activity of the compound of the present disclosure on HepG2 cell line**

| Compound No. | IC₅₀ (nM) |
|---|---|
| Compound of formula (I) | 7.4 |

Conclusion: The compound of formula (I) has excellent anti-proliferative activity on HepG2 with high expression of AKR1C3.

### Experimental example 2: Anti-proliferative activity of compound of formula (I) on Hep3B cell line

### Experimental materials:

EMEM medium, penicillin/streptomycin antibiotics were purchased from Wisent, and fetal bovine serum was purchased from Biosera. CellTiter-Glo (chemiluminescent cell viability assay reagent) reagent was purchased from Promega. The Hep3B cell line was purchased from the Cell Bank of the Chinese Academy of Sciences. Nivo multimode microplate reader (PerkinElmer).

### Experimental methods:

Hep3B cells (liver cancer) were seeded in a white 96-well plate with 80 µL of cell suspension per well containing 3000 Hep3B cells. The cell plate was incubated overnight in a carbon dioxide incubator. The compound to be tested was 5-fold diluted to the ninth concentration with a multi-channel pipette, that is, the compound to be tested was diluted from 2 mM to 5.12 nM, and an experiment for the sample assayed in duplicate was set up. 78 µL of culture medium was added to the middle plate. According to the corresponding position, 2 µL per well of the gradient-diluted compound was transferred to the middle plate, then 20 µL per well was transferred to the cell plate after mixing well. The concentration of the compound transferred to the cell plate ranged from 10 µM to 0.0256 nM. The cell plate was incubated in the carbon dioxide incubator for 3 days. Another cell plate was prepared, and the signal value as the maximum value (Max value in the following equation) was read on the day of drug addition to participate in data analysis. 25 µL of chemiluminescent cell viability assay reagent was added to each well of the cell plate, and the plate was incubated at room temperature for 10 minutes to stabilize the luminescent signal. A multimode microplate reader was used for reading. 25 µL of chemiluminescent cell viability assay reagent was added to each well of the cell plate, and the plate was incubated at room temperature for 10 minutes to stabilize the luminescent signal. A multimode microplate reader was used for reading.

### Data analysis:

The equation (Sample - Min)/(Max - Min) * 100% was used to convert the raw data into the inhibition rate, and the value of IC₅₀ could be obtained by curve fitting with four parameters ("log(inhibitor) vs. response -- Variable slope" mode in GraphPad Prism). Table 6 provides the inhibitory activity of the compound of formula (I) on the proliferation of Hep3B cells.

**Table 6: Data of anti-proliferative activity of the compound of formula (I) on Hep3B cell line**

| Compound No. | IC₅₀ (nM) |
|---|---|
| Compound of formula (I) | >10,000 |

Conclusion: The compound of formula (I) has no anti-proliferative activity on Hep3B with low expression of AKR1C3, showing high selectivity.

**Experimental embodiment 3:** *In vivo* pharmacodynamics study of compound of formula (I) on orthotopic xenograft tumor model of human liver cancer HepG2

### Experimental purpose:

In this experiment, orthotopic xenograft tumor model of HepG2 in a nude mouse was used to evaluate the anti-tumor effect of the compound.

### Experimental materials:

Female Balb/C nude mice, 6 to 8 weeks old, weighing 18 to 22 g, fetal bovine serum (PBS), EMEM medium (Cat. No. 30-2003), phosphate buffer, Antibiotic-Antimycotic (Cat. No. 15240-062), Matrigel, and trypsin.

### Experimental methods and steps

1. Preparation of cell culture: HepG2-luc cells were cultured in monolayer *in vitro.* The culture conditions were that 10% heat-inactivated fetal bovine serum was added to EMEM medium and the cells were cultured in an incubator containing 5% CO₂ at 37°C. Digestion with trypsin-EDTA was performed twice a week for passaging. When the cell saturation was 80% to 90%, the cells were digested with trypsin-EDTA, counted, and resuspended in PBS and Matrigel (PBS: Matrigel = 1:1), and the density was 166.67 × 10⁶ cells/mL.
2. Tumor cell inoculation and grouping: Animals were anesthetized by intramuscular injection of 60 mg/kg Zoletil 50 and 1.5 mg/kg xylazine. When the animals were under deep anesthesia, the animals were properly fixed, and the abdomen skin was cleaned with 75% alcohol cotton balls. An incision of about 10 mm was cut with surgery, and 0.03 mL (PBS: Matrigel = 1:1) of HepG2-luc cells was orthotopically inoculated on the left lobe of the liver of each mouse, and then the incision on the muscle layer was sutured with absorbable catgut, and the incision on the skin was sutured with a stapler. After surgery, the animals were kept warm on a warming blanket until they woke up. To relieve the pain of the animals, 2 mg/kg of meloxicam (administered subcutaneously once a day) would be administered for 3 consecutive days after surgery. 15 animals were randomly selected to detect the growth of the signal. When the signal began to rise, animals were randomly divided into groups according to the value of the bioluminescence signal, and the administration of the drug was started. The detailed treatment regimen is shown in Table 7.

**Table 7: Grouping and administration regimen of experimental animals**

| Group | Number of animals | Drug | Dose (mg/kg) | Administration volume (µL/g)² | Route of administration | Frequency of administration |
|---|---|---|---|---|---|---|
| 1 | 6 | Blank group | -- | 10 | Intravenous injection (IV) | Day 0, Day 7, Day 14 |
| 2 | 6 | Compound of formula (I) | 1 | 10 | Intravenous injection (IV) | Day 0, Day 7, Day 14 |
| 3 | 6 | Compound of formula (I) | 3 | 10 | Intravenous injection (IV) | Day 0, Day 7, Day 14 |

### 3. Experimental indicator

The experimental indicator is whether the tumor growth can be delayed or whether the tumor can be cured. After tumor inoculation, the bioluminescence signal and animal weight were detected once a week, which continued until the end of the observation period. The bioluminescence signal value can be used to calculate T/C (wherein T is the administration group, and C is the average intensity value of bioluminescence of the blank control group at the set time). Calculation formula of tumor growth inhibition rate (TGI): TGI (%) = [1 - (Tᵢ - T₀) / (Vᵢ - V₀)] × 100, wherein Tᵢ is the average intensity of bioluminescence of the treatment group at the set time; T₀ is the average intensity of bioluminescence at the starting point of administration. Vᵢ is the average intensity of bioluminescence of the blank control group at the set time; V₀ is the average intensity of bioluminescence at the starting point of administration.

### 4. Inhibitory effect of the compound on the growth of subcutaneously transplanted tumors of HepG2 liver cancer in nude mice

In this experiment, the efficacy of the compound of formula (I) in the orthotopic xenograft tumor model of HepG2 liver cancer was evaluated. After 21 days of administration, the compound of formula (I) had a significant inhibitory effect on tumor growth at a dose of 1 mg/kg with p < 0.05 compared with the vehicle control group. With the dose of the compound of formula (I) increased to 3 mg/kg, the anti-tumor effect was significantly enhanced.

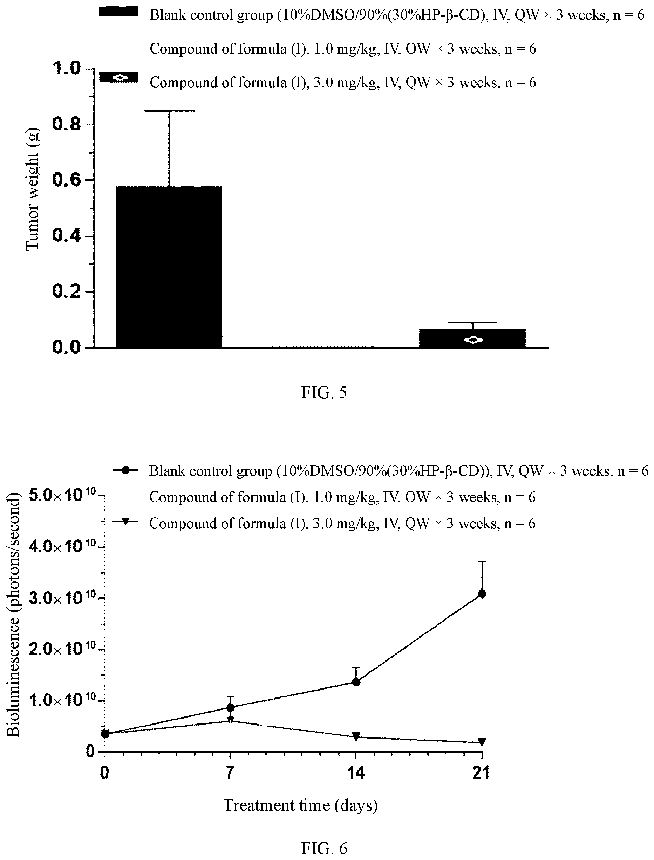

Experimental results: See FIGS. 5 and 6 and Tables 8, 9, and 10.

**Table 8: Anti-tumor effect of the compound of formula (I) on orthotopic xenograft tumor model of HepG2**

| Group | Bioluminescence (photons/second)^{a} (Day 1) | Bioluminescence (photons/second)^{a} (Day 21) | RBL (Day 21) | TGI (%) (Day 21) | T/C (%) (Day 21) |
|---|---|---|---|---|---|
| 1 | 3.51E+09 ± 7.27E+08 | 3.09E+10 ± 6.17E+09 | 9.78 ± 1.67 | -- | -- |
| 2 | 3.51E+09 ± 7.41E+08 | 4.35E+09 ± 2.19E+09 | 0.98 ± 0.31 | 96.97 | 10.04 |
| 3 | 3.51E+09 ± 7.96E+08 | 1.81E+09 ± 7.52E+08 | 0.41 ± 0.12 | 106.21 | 4.22 |

| | | | | | |
|---|---|---|---|---|---|
| Note: a. Mean ± SEM, n = 6. | | | | | |

**Table 9: p values for comparison of relative tumor signal growth values (RBL) between groups in the xenograft tumor model of HepG2**

| Group | Group 1 | Group 2 | Group 3 |
|---|---|---|---|
| 1 | N/A | 0.029 | 0.024 |
| 2 | 0.029 | N/A | 0.0682 |
| 3 | 0.024 | 0.682 | N/A |

| | | | |
|---|---|---|---|
| Note: The *p* values were obtained by using one-way ANOVA to analyze tumor volume relative values (RBL). The F values were significantly different between groups (p < 0.001), and the Games-Howell test was used. | | | |

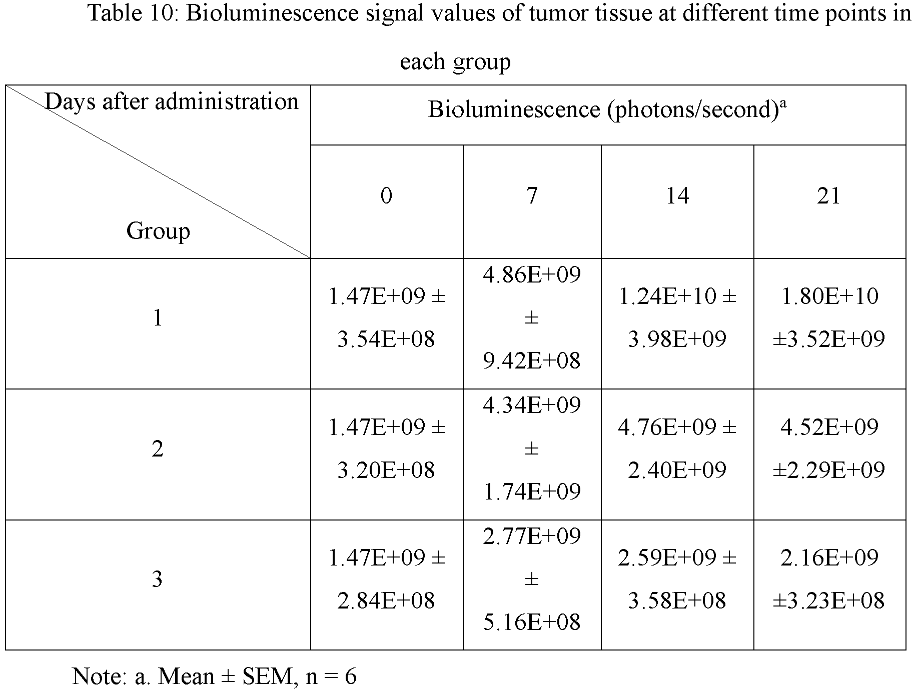

**Table 11: Tumor weight in each group**

| Group | Tumor weight (g)^{a} | T/C_{weight}^{b} | *p* value^{c} |
|---|---|---|---|
| | (Day 21) | (%) | |
| 1 | 0.569 ± 0.115 | -- | -- |
| 2 | 0.090 ± 0.036 | 15.80 | 0.072 |
| 3 | 0.058 ± 0.013 | 10.23 | 0.059 |

| | | | |
|---|---|---|---|
| Note: a. Mean ± SEM, n = 6. b. Tumor growth inhibition was calculated by T/C_{weight} = TWₜᵣₑₐₜₘₑₙₜ / TW_{vehicle}. c. The *p* values were obtained by analyzing the tumor weight using one-way ANOVA between the vehicle group and the treatment groups. The F values were significantly different (p < 0.001), and the Games-Howell test was used for analysis. | | | |

### 5. Changes in weight

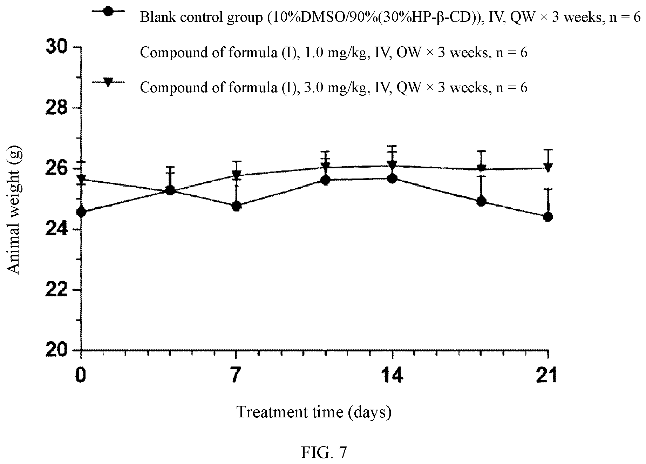

In this model, the weight of animals in all treatment groups did not fluctuate greatly, and the decrease of average weight of all animals did not exceed 5%, as shown in FIG. 7.

6. Conclusion: The compound of formula (I) has a significant inhibitory effect on tumor growth, and the animal weight in the administration group does not decrease significantly, showing good safety.

## Claims

1. A crystal form A of a compound of formula (I) 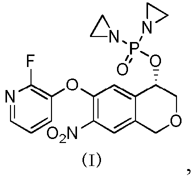 wherein the crystal form A of the compound of formula (I) has an X-ray powder diffraction pattern comprising characteristic diffraction peaks at the following 2θ angles: 8.25 ± 0.20°, 19.21 ± 0.20°, and 21.76 ± 0.20° using Cu-Kα radiation.

2. The crystal form A of the compound of formula (I) according to claim 1, wherein the X-ray powder diffraction pattern thereof comprises characteristic diffraction peaks at the following 2θ angles: 8.25 ± 0.20°, 13.27 ± 0.20°, 15.19 ± 0.20°, 16.43 ± 0.20°, 17.94 ± 0.20°, 19.21 ± 0.20°, and 21.76 ± 0.20°.

3. The crystal form A of the compound of formula (I) according to claim 1, wherein the X-ray powder diffraction pattern thereof comprises characteristic diffraction peaks at the following 2θ angles: 8.25 ± 0.20°, 15.19 ± 0.20°, 16.43 ± 0.20°, 17.94 ± 0.20°, 19.21 ± 0.20°, 21.76 ± 0.20°, 23.56 ± 0.20°, and 26.66 ± 0.20°.

4. The crystal form A of the compound of formula (I) according to claim 3, wherein the X-ray powder diffraction pattern thereof comprises characteristic diffraction peaks at the following 2θ angles: 8.25°, 12.56°, 12.89°, 13.27°, 15.19°, 16.01°, 16.43°, 17.60°, 17.94°, 19.21°, 19.91°, 20.21°, 20.52°, 21.25°, 21.76°, 23.02°, 23.56°, 24.71°, 25.12°, 25.88°, 26.66°, 27.10°, 27.43°, 27.84°, 28.38°, 29.13°, 30.01°, 30.83°, 31.69°, 32.26°, 33.35°, 35.01°, 35.52°, 36.51°, 37.70°, and 39.24°.

5. The crystal form A of the compound of formula (I) according to claim 4, wherein the crystal form A of the compound of formula (I) has an X-ray powder diffraction pattern basically as shown in FIG. 1.

6. The crystal form A of the compound of formula (I) according to any one of claims 1 to 5, wherein the crystal form A of the compound of formula (I) has a differential scanning calorimetry curve comprising an endothermic peak at 89.2 ± 3°C.

7. The crystal form A of the compound of formula (I) according to claim 6, wherein the crystal form A of the compound of formula (I) has a DSC pattern basically as shown in FIG. 2.

8. The crystal form A of the compound of formula (I) according to any one of claims 1 to 5, wherein the crystal form A of the compound of formula (I) has a thermogravimetric analysis curve with a weight loss of 1.03% at 100 ± 3°C.

9. The crystal form A of the compound of formula (I) according to claim 8, wherein the crystal form A of the compound of formula (I) has a TGA pattern basically as shown in FIG. 3.

10. The crystal form A of the compound of formula (I) according to any one of claims 1 to 9 for use in treating liver cancer, prostate cancer, pancreatic cancer, and/or T-cell acute lymphoblastic leukemia.

## Patentansprüche

1. Kristallform A einer Verbindung der Formel (I), 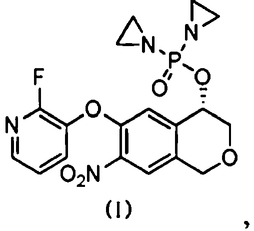 wobei die Kristallform A der Verbindung der Formel (I) ein Röntgenpulverdiffraktogramm aufweist, das charakteristische Diffraktionspeaks bei den folgenden 2θ-Winkeln umfasst: 8,25 ± 0,20°, 19,21 ± 0,20° und 21,76 ± 0,20° unter Verwendung von CuKα-Strahlung.

2. Kristallform A der Verbindung der Formel (I) nach Anspruch 1, wobei das Röntgenpulverdiffraktogramm davon charakteristische Diffraktionspeaks bei den folgenden 2θ-Winkeln umfasst: 8,25 ± 0,20°, 13,27 ± 0,20°, 15,19 ± 0,20°, 16,43 ± 0,20°, 17,94 ± 0,20°, 19,21 ± 0,20° und 21,76 ± 0,20°.

3. Kristallform A der Verbindung der Formel (I) nach Anspruch 1, wobei das Röntgenpulverdiffraktogramm davon charakteristische Diffraktionspeaks bei den folgenden 2θ-Winkeln umfasst: 8,25 ± 0,20°, 15,19 ± 0,20°, 16,43 ± 0,20°, 17,94 ± 0,20°, 19,21 ± 0,20°, 21,76 ± 0,20°, 23,56 ± 0,20° und 26,66 ± 0,20°.

4. Kristallform A der Verbindung der Formel (I) nach Anspruch 3, wobei das Röntgenpulverdiffraktogramm davon charakteristische Diffraktionspeaks bei den folgenden 2θ-Winkeln umfasst: 8,25°, 12,56°, 12,89°, 13,27°, 15,19°, 16,01°, 16,43°, 17,60°, 17,94°, 19,21°, 19,91°, 20,21°, 20,52°, 21,25°, 21,76°, 23,02°, 23,56°, 24,71°, 25,12°, 25,88°, 26,66°, 27,10°, 27,43°, 27,84°, 28,38°, 29,13°, 30,01°, 30,83°, 31,69°, 32,26°, 33,35°, 35,01°, 35,52°, 36,51°, 37,70° und 39,24°.

5. Kristallform A der Verbindung der Formel (I) nach Anspruch 4, wobei die Kristallform A der Verbindung der Formel (I) ein Röntgenpulverdiffraktogramm aufweist, das im Wesentlichen wie in Figur 1 dargestellt ist.

6. Kristallform A der Verbindung der Formel (I) nach einem der Ansprüche 1 bis 5, wobei die Kristallform A der Verbindung der Formel (I) eine Differential-Scanning-Kalorimetrie-Kurve aufweist, umfassend einen endothermen Peak bei 89,2 ± 3 °C.

7. Kristallform A der Verbindung der Formel (I) nach Anspruch 6, wobei die Kristallform A der Verbindung der Formel (I) ein DSC-Muster aufweist, das im Wesentlichen wie in Figur 2 dargestellt ist.

8. Kristallform A der Verbindung der Formel (I) nach einem der Ansprüche 1 bis 5, wobei die Kristallform A der Verbindung der Formel (I) eine thermogravimetrische Analyse-Kurve mit einem Gewichtsverlust von 1,03 % bei 100 ± 3 °C aufweist.

9. Kristallform A der Verbindung der Formel (I) nach Anspruch 8, wobei die Kristallform A der Verbindung der Formel (I) ein TGA-Muster aufweist, das im Wesentlichen wie in Figur 3 dargestellt ist.

10. Kristallform A der Verbindung der Formel (I) nach einem der Ansprüche 1 bis 9 zur Verwendung bei der Behandlung von Leberkrebs, Prostatakrebs, Bauchspeicheldrüsenkrebs und/oder T-Zell-akuter lymphoblastischer Leukämie.

## Revendications

1. Forme cristalline A d'un composé de formule (I), 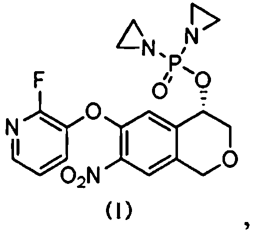 dans laquelle la forme cristalline A du composé de formule (I) présente un diagramme de diffraction des rayons X sur poudre comprenant des pics de diffraction caractéristiques aux angles de diffraction 2θ suivants : 8,25 ± 0,20°, 19,21 ± 0,20°, et 21,76 ± 0,20° en utilisant le rayonnement Cu-Kα.

2. Forme cristalline A du composé de formule (I) selon la revendication 1, dans laquelle le diagramme de diffraction des rayons X sur poudre de celle-ci comprend des pics de diffraction caractéristiques aux angles de diffraction 2θ suivants : 8,25 ± 0,20°, 13,27 ± 0,20°, 15,19 ± 0,20°, 16,43 ± 0,20°, 17,94 ± 0,20°, 19,21 ± 0,20°, et 21,76 ± 0,20°.

3. Forme cristalline A du composé de formule (I) selon la revendication 1, dans laquelle le diagramme de diffraction des rayons X sur poudre de celle-ci comprend des pics de diffraction caractéristiques aux angles de diffraction 2θ suivants : 8,25 ± 0,20°, 15,19 ± 0,20°, 16,43 ± 0,20°, 17,94 ± 0,20°, 19,21 ± 0,20°, 21,76 ± 0,20°, 23,56 ± 0,20°, et 26,66 ± 0,20°.

4. Forme cristalline A du composé de formule (I) selon la revendication 3, dans laquelle le diagramme de diffraction des rayons X sur poudre de celle-ci comprend des pics de diffraction caractéristiques aux angles de diffraction 2θ suivants : 8,25°, 12,56°, 12,89°, 13,27°, 15,19°, 16,01°, 16,43°, 17,60°, 17,94°, 19,21°, 19,91°, 20,21 °, 20,52°, 21,25°, 21,76°, 23,02°, 23,56°, 24,71°, 25,12°, 25,88°, 26,66°, 27,10°, 27,43°, 27,84°, 28,38°, 29,13°, 30,01 °, 30,83°, 31,69°, 32,26°, 33,35°, 35,01°, 35,52°, 36,51°, 37,70°, et 39,24°.

5. Forme cristalline A du composé de formule (I) selon la revendication 4, dans laquelle la forme cristalline A du composé de formule (I) présente un diagramme de diffraction des rayons X sur poudre sensiblement conforme à celui représenté sur la figure 1.

6. Forme cristalline A du composé de formule (I) selon l'une quelconque des revendications 1 à 5, dans laquelle la forme cristalline A du composé de formule (I) présente une courbe de calorimétrie différentielle à balayage (DSC) comprenant un pic endothermique à 89,2 ± 3 °C.

7. Forme cristalline A du composé de formule (I) selon la revendication 6, dans laquelle la forme cristalline A du composé de formule (I) présente un profil DSC sensiblement conforme à celui représenté sur la figure 2.

8. Forme cristalline A du composé de formule (I) selon l'une quelconque des revendications 1 à 5, dans laquelle la forme cristalline A du composé de formule (I) présente une courbe d'analyse thermogravimétrique (TGA) avec une perte de poids de 1,03 % à 100 ± 3 °C.

9. Forme cristalline A du composé de formule (I) selon la revendication 8, dans laquelle la forme cristalline A du composé de formule (I) présente un profil TGA sensiblement conforme à celui représenté sur la figure 3.

10. Forme cristalline A du composé de formule (I) selon l'une quelconque des revendications 1 à 9 pour une utilisation dans le traitement du cancer du foie, du cancer de la prostate, du cancer du pancréas, et/ou de la leucémie aiguë lymphoblastique à cellules T.
